# EUROPÄISCHE PATENTSCHRIFT

(11) **EP 2 306 963 B1**
(45) Veröffentlichungstag und Bekanntmachung des Hinweises auf die Patenterteilung: **28.03.2018**
(21) Anmeldenummer: 09779666.8
(22) Anmeldetag: 08.06.2009
(51) Int. Cl.: A61Q 5/08, A61Q 5/06, A61K 8/37, A61K 8/34, A61K 8/46, A61K 8/49, A61K 8/60

(54) **MATTIERUNGSADDITIV FÜR BLONDIERUNGEN**
MATTING ADDITIVE FOR BLEACHING
ADDITIF DE MATAGE POUR DES DÉCOLORATIONS

(30) Priorität: 08.08.2008 DE 102008036957
(43) Veröffentlichungstag der Anmeldung: 13.04.2011
(73) Patentinhaber: Henkel AG & Co. KGaA, 40589 Düsseldorf (DE)
(72) Erfinder: MANNECK, Hartmut, 23860 Klein Wesenberg (DE)
(86) Internationale Anmeldenummer: PCT/EP2009/057013
(87) Internationale Veröffentlichungsnummer: WO 2010/015441

(56) Entgegenhaltungen:
- EP-A- 1 759 684
- CA-A1- 2 613 049
- DE-A1- 19 745 293
- DE-U1- 20 303 559
- US-A1- 2004 143 910
- US-A1- 2005 196 367

## Beschreibung

Gegenstand der vorliegenden Anmeldung sind Additive zur Mattierung von Blondierungen keratinischer Fasern, insbesondere menschlicher Haare, sowie Mittel zum Blondieren keratinischer Fasern ohne unerwünschte Farbverschiebung in gelbe bis rötliche Nuancen. Weiterhin werden eine Verwendung zur Blondierung keratinischer Fasern ohne unerwünschte Farbverschiebung und ein Verfahren zur Blondierung unter Einsatz des erfindungsgemäßen Mittels beschrieben. Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Dabei können Dauerwell- und andere die Haarform verändernde Verfahren nahezu unabhängig vom Typ der zu behandelnden Haare eingesetzt werden. Dagegen sind Färbe- und insbesondere Blondierverfahren auf bestimmte Ausgangshaarfarben begrenzt. So eignen sich für aufhellende Verfahren, die so genannten Blondierverfahren, im Wesentlichen nur dunkelblonde oder hellere Haare. Insbesondere für Blondierungen von dunkleren Ausgangshaarfarben besteht weiterhin ein Bedarf an leistungsfähigen Methoden. Die Grundlagen der Blondierverfahren sind dem Fachmann bekannt und in einschlägigen Monographien, z. B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.
Zum Blondieren menschlicher Haare werden üblicherweise feste oder pastenförmige Zubereitungen mit festen Oxidationsmitteln unmittelbar vor der Anwendung mit einer verdünnten Wasserstoffperoxid-Lösung vermischt. Diese Mischung wird dann auf das Haar aufgebracht und nach einer bestimmten Einwirkzeit wieder ausgespült. Die Einwirkungsdauer auf dem Haar zur Erzielung einer vollständigen Ausfärbung Aufhellung liegt zwischen etwa 30 und 60 Minuten. Es ist naheliegend, dass bei den Benutzern dieser Blondiermittel ein Bedürfnis besteht, diese Einwirkungszeit zu verringern.
Um eine ausreichenden Blondierwirkung zu erzielen, sind derartige Mittel üblicherweise stark alkalisch eingestellt, der pH-Wert liegt dabei zwischen 9 und 10,5. Derart hohe pH-Werte sind erforderlich, um eine Öffnung der äußeren Schuppenschicht (Cuticula) zu gewährleisten und somit eine Penetration der aktiven Spezies (Wasserstoffperoxid) ins Haar zu ermöglichen. Als Alkalisierungsmittel wird üblicherweise Ammoniak eingesetzt, der allerdings den bekannten Nachteil des unangenehmen Geruchs und eventueller Reizung aufweist.

In der Vergangenheit wurden häufig auch alternative Alkalisierungsmittel eingesetzt, die zwar in Abmischung mit Ammoniak selber akzeptable Blondierleistungen ergaben, diesen aber nicht vollständig ersetzen konnten. Es blieb daher bisher der Wunsch bestehen, die Ammoniakkonzentration zu reduzieren, ohne Abstriche bei der Blondierleistung der Mittel in Kauf nehmen zu müssen.

Um beim Bleichvorgang große Farbunterschiede zu erzielen, also insbesondere dunkle oder schwarze Haare zu entfärben, werden neben den bereits erwähnten Wasserstoffperoxidzubereitungen zumeist Bleichaktivatoren wie beispielsweise Persalze als zusätzliche Peroxidquellen, und/oder Carbonate als zusätzliche Alkalisierungsmittel benötigt.

Daher stellt die Blondierung von Haaren mit dunkler Ausgangsfarbe eine besondere Herausforderung dar. Die natürliche Haarfarbe wird durch Melamine in der Cortex der Haarfaser bestimmt, wobei das Verhältnis von der zwei Pigmentklassen, Eumelanine mit bräunlich-schwarzen Tönen und Pheomelanine mit rötlich-orangen Tönen, die eigentliche Haarfarbe bestimmt. Üblicherweise werden beim Blondierprozess die natürlichen Melanin-Farbstoffe durch oxidative Einwirkung zerstört und so eine Entfärbung der Fasern erreicht. Aufgrund der unterschiedlichen oxidativen Zerstörungsrate der verschiedenen Melaninpigmentklassen werden Haare jedoch nicht gleichmäßig entfärbt. In dunkleren Fasern mit hohem Melaningehalt verbleibt zumeist ein bestimmter Anteil an Farbstoffen, der sich häufig in gelblichen bis rötlichen Nuancen niederschlägt. Daher kommt es insbesondere beim Blondieren von dunkleren Haaren zu einer Farbverschiebung in Richtung warmer Töne. Üblicherweise sind solche Farbverschiebungen in Richtung warmer Töne bei dem Anwender unerwünscht. Daher wird dieser Farbverschiebung zumeist durch eine Tönung mit der entsprechenden Komplementärfarbe gemäß der Farbenlehre entgegengewirkt. Ziel ist dabei ein silbrig-kühler Eindruck des Bleichergebnisses. Der Fachmann spricht in diesem Zusammenhang von einer Mattierung. Je nach Ausgangshaarfarbe muss dabei eine angepasste Tönungsmittelmischung eingesetzt werden, um entsprechend rötlichere Farbverschiebungen durch grünlichere Tönungsmittel oder gelblichere Farbverschiebungen durch eher violette Tönungsmittel auszugleichen.

Unter den aggressiven Bedingungen (starke Oxidationsmittel, hoher pH-Wert) der aufhellenden Zubereitung sind die üblicherweise eingesetzten Tönungsmittel zumeist nicht ausreichend stabil. Daher müssen Blondierschritt und Tönungsschritt üblicherweise nacheinander erfolgen. Aus Gründen des Anwendungskomforts ist aber eine einstufige Blondierung, die bereits den mattierenden Farbausgleich der unerwünschten Warmtöne beinhaltet, besonders vorteilhaft. Daher besteht ein Bedürfnis nach Blondiermitteln, die durch Zusatz von stabilen Tönungskomponenten in die Anwendungszubereitung einen guten Farbausgleich und damit eine hohe Mattierung erzielen.

Im Rahmen ihrer Untersuchungen hat die Anmelderin nun überraschend gefunden, dass die Mischung von roten und blauen direktziehenden Farbstoffen in einem bestimmten Gewichtsverhältnis in Kombination mit einer nichtionischen Komponente mit einem HLB von kleiner oder gleich 8,0 sich hervorragend als stabiles Mattierungsadditiv für Blondier- und/oder Aufhellmittel für keratinische Fasern, insbesondere menschliche Haare, eignet.

Ein erster Gegenstand der vorliegenden Erfindung ist daher ein Mittel gemäß Anspruch 1. Unter keratinhaltigen bzw. keratinischen Fasern werden erfindungsgemäß Pelze, Wolle, Federn und insbesondere menschliche Haare verstanden. Obwohl die erfindungsgemäßen Mittel in erster Linie zum Färben und/oder Aufhellen von keratinhaltigen Fasern geeignet sind, steht prinzipiell einer Verwendung auch auf anderen Gebieten nichts entgegen.
Die erfindungsgemäßen Mittel enthalten die Wirkstoffe in einem kosmetischen Träger. Dieser kosmetische Träger ist im Sinne der Erfindung ist wässrig, alkoholisch oder wässrig-alkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als erstes zwingendes Merkmal enthält das erfindungsgemäße Mittel eine bestimmte nichtionische Komponente mit einem HLB-Wert von kleiner oder gleich 8,0. Nichtionisch im Sinne der Erfindung bedeutet dabei, dass die Komponente in wässriger Lösung keine Ionen bildet.
Ohne an eine bestimmte Theorie gebunden zu sein, vermutet die Anmelderin, dass durch hydrophobe Wechselwirkungen zwischen der nichtionischen Komponente und den direktziehenden Farbstoffen ein Schutz dieser Farbstoffe unter den aggressiven Bedingungen der Blondiermittel ermöglicht wird.

Der HLB-Wert ist ein von Griffin (1950) eingeführtes Maß für die Wasser- bzw. Öllöslichkeit von Verbindungen, wobei Verbindungen mit niedrigen HLB-Werten hydrophobere Eigenschaften besitzen als Verbindungen mit hohen HLB-Werten. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen. Erfindungsgemäße Mittel sind dadurch gekennzeichnet, dass sie als nichtionische Komponente mit einem HLB-Wert von kleiner oder gleich 8,0 mindestens einen Fettalkohol mit 12, 14, 16 oder 18 Kohlenstoffatomen in der Alkylkette enthalten. Es ist dabei bevorzugt, wenn die nichtionische Komponente mit einem HLB-Wert von kleiner oder gleich 8,0 zu 1,0 Gew.-% bis 50 Gew.-%, insbesondere zu 2,0 Gew.-% bis 25 Gew.-% und ganz besonders bevorzugt zu 3,0 Gew.-% bis 15 Gew.-%, jeweils bezogen auf das Gesamtgewicht des anwendungsbereiten Mittels, im erfindungsgemäßen Mittel vorliegt.
Als weiteren zwingenden Bestandteil enthält das erfindungsgemäße Mittel eine Kombination aus mindestens einem bestimmten blauen direktziehenden Farbstoff und einem bestimmten roten direktziehenden Farbstoff, wobei das Gewichtsverhältnis zwischen der Summe aller blauen direktziehenden Farbstoffe und der Summe aller roten direktziehenden Farbstoffe einen Wert von größer oder gleich 1 besitzt.
Dadurch ist es möglich, unerwünschte Farbverschiebungen in Richtung rosa-/rose-farbene Nuancen zu vermieden.
Erfindungsgemäß bevorzugte Mittel sind dadurch gekennzeichnet, dass das Gesamtgewicht aller blauen direktziehenden Farbstoffe größer als das Gesamtgewicht aller roten direktziehenden Farbstoffe ist.
Bevorzugte Mittel sind daher dadurch gekennzeichnet, das Gewichtsverhältnis zwischen der Summe aller blauen direktziehenden Farbstoffe und der Summe aller roten direktziehenden Farbstoffe einen Wert von 1 bis 100, bevorzugt von 1,5 bis 10 und besonders bevorzugt von 2 bis 4 besitzt. Das Mittel enthält als blauen direktziehenden Farbstoff mindestens eine Verbindung gemäß Formel (I) und/oder eines ihrer physiologisch verträglichen Salze 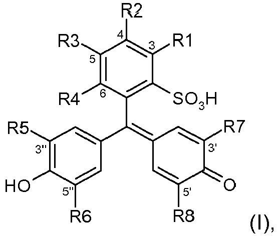 worin
R1, R2, R3, R4, R5, R6, R7 und R8 jeweils für Brom steht. Diese Verbindung ist auch unter dem Namen Tetrabromphenolblau bekannt. Je nach pH-Wert der Mittel liegt die Verbindung gemäß Formel (I) in deprotonierter Form vor als eines ihrer physiologisch verträglichen Salze. Physiologisch verträgliche Salze sind dabei Alkalimetall-, Erdalkalimetall- oder Ammonium-Salze, wobei sowohl Ammonium selbst wie auch primär-, sekundär, tertiär oder quaternär substituierte Ammonium-Salze eingesetzt werden können. Hierzu zählen beispielsweise die Salze der Kationen 2-Hydroxyethylammonium, Di(2-hydroxyethyl)-ammonium, Tri(2-hydroxyethyl)ammonium, Triethylammonium und Tetraethylammonium. Bevorzugte Salze der Verbindung gemäß Formel (I) sind Natrium-, Kalium-, Magnesium-, Calcium- und Ammonium-Salze.
Weiterhin liegen Verbindungen gemäß Formel (I) pH-Wert abhängig im tautomeren Gleichgewicht mit ihrer cyclisierten Form gemäß Formel (la) und/oder einem ihrer physiologisch verträglichen Salze. 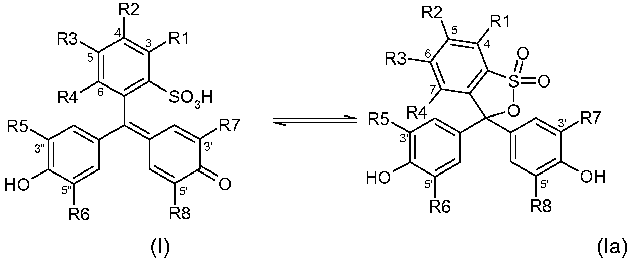 Beide Strukturformeln (I) und (la) geben daher erfindungsgemäß dieselbe Verbindung wieder. Das Mittel enthält als roten direktziehenden Farbstoff mindestens eine Verbindung gemäß Formel (II) und/oder eines ihrer physiologisch verträglichen Salze 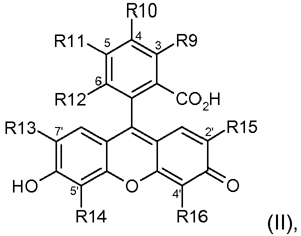 worin
R9, R10, R11 und R12 für Chlor und R13, R14, R15 und R16 für Brom (bekannt als Acid Red 92) stehen. Diese Verbindung ist auch unter dem Namen D&C RED No. 28 oder Phloxin B bekannt. Je nach pH-Wert der Mittel liegt die Verbindung gemäß Formel (II) in deprotonierter Form vor als eines ihrer physiologisch verträglichen Salze. Physiologisch verträgliche Salze sind dabei Alkalimetall-, Erdalkalimetall- oder Ammonium-Salze, wobei sowohl Ammonium selbst wie auch primär-, sekundär, tertiär oder quaternär substituierte Ammonium-Salze eingesetzt werden können. Hierzu zählen beispielsweise die Salze der Kationen 2-Hydroxyethylammonium, Di(2-hydroxyethyl)-ammonium, Tri(2-hydroxyethyl)ammonium, Triethylammonium und Tetraethylammonium. Bevorzugte Salze der Verbindung gemäß Formel (II) sind Natrium-, Kalium-, Magnesium-, Calcium- und Ammonium-Salze.
Weiterhin liegen Verbindungen gemäß Formel (II) pH-Wert abhängig im tautomeren Gleichgewicht mit ihrer cyclisierten Form gemäß Formel (IIa) und/oder einem ihrer physiologisch verträglichen Salze. 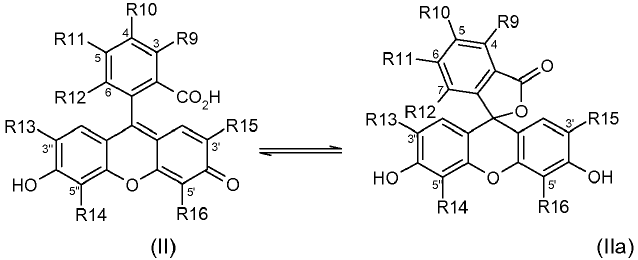 Beide Strukturformeln (II) und (IIa) geben daher erfindungsgemäß dieselbe Verbindung wieder.

Es kann erfindungsgemäß weiterhin bevorzugt sein, wenn das Mittel weitere direktziehende Farbstoffe enthält. Besonders bevorzugt enthält das Mittel als weiteren direktziehenden Farbstoff einen gelben und/oder orangen Farbstoff.
Dies ist insbesondere dann vorteilhaft, wenn unerwünschte rötliche Farbverschiebungen beim Blondierprozess auftreten.
Bevorzugt werden die gelben Farbstoffe ausgewählt aus geeigneten gelben Nitrofarbstoffe, wie1,2-Diamino-4-nitrobenzol (C.I. 76,020), 1-[(2-Hydroxyethyl)amino]-2-nitrobenzol (HC Yellow 2), 1-(2-Hydroxyethoxy)-2-[(2-hydroxyethyl)amino]-5-nitrobenzol (HC Yellow 4), 1-Amino-2-[(2-hydroxyethyl)amino]-5-nitrobenzol (HC Yellow 5), 4-[(2,3-Dihydroxypropyl)amino]-3-nitro-1-trifluormethyl-benzol (HC Yellow 6), 2-[Di(2-hydroxyethyl)amino]-5-nitrophenol, 2-[(2-Hydroxyethyl)amino]-1-methoxy-5-nitrobenzol, 2-Amino-3-nitrophenol, 2-Amino-4-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, 3-[(2-Aminoethyl)amino]-1-methoxy-4-nitrobenzol-hydrochlorid (HC Yellow 9), 1-Chlor-2,4-bis[(2-hydroxyethyl)amino]-5-nitrobenzol (HC Yellow 10), 2-[(2-Hydroxyethyl)amino]-5-nitrophenol (HC Yellow 11), 1-[(2'-Ureidoethyl)amino]-4-nitrobenzol, 1-Amino-4-[(2-aminoethyl)-amino]-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow 12), 4-[(2-Hydroxyethyl)amino]-3-nitro-1-trifluor-methylbenzol (HC Yellow 13), 4-[(2-Hydroxyethyl)-amino]-3-nitro-benzonitril (HC Yellow 14), 4-[(2-Hydroxyethyl)amino]-3-nitro-benzamid (HC Yellow 15) 3-[(2-Hydroxyethyl)amino]-4-methyl-1-nitrobenzol, 4-Chlor-3-[(2-hydroxyethyl)amino]-1-nitrobenzol.

Geeignete gelbe oder orange Chinonfarbstoffe sind insbesondere 2-[(2-Aminoethyl)amino]-9,10-anthrachinon (HC Orange 5) und 2-Hydroxy-1,4-naphthochinon (C.I. 75,480, Natural Orange 6). Auch neutrale, gelbe oder orange Azofarbstoffe können erfindungsgemäß vorteilhaft eingesetzt werden, insbesondere 4-[(4-Aminophenyl)azo]-1-[di(2-hydroxyethyl)amino]-3-methylbenzol (HC Yellow 7), 2,6-Diamino-3-[(pyridin-3-yl)azo]pyridin, 2-{[4-(Acetylamino)phenyl]azo}-4-methylphenol (C.I. 11855; Disperse Yellow 3), 4-[(4-Nitrophenyl)azo]anilin (C.I. 11,005; Disperse Orange 3).

Als gelbe oder orange, anionische direktziehende Farbstoffe eignen sich insbesondere 6-Hydroxy-5-[(4-sulfophenyl)azo]-2-naphthalinsulfonsäuredinatriumsalz (C.I. 15,985; Food Yellow No. 3; FD&C Yellow No. 6), 2,4-Dinitro-1-naphthol-7-sulfonsäure-dinatriumsalz (C.I. 10,316; Acid Yellow 1; Food Yellow No. 1), 2-(Indan-1,3-dion-2-yl)chinolin-x,x-sulfonsäure (Gemisch aus Mono- und Disulfonsäure) (C.I. 47,005; D&C Yellow No. 10; Food Yellow No. 13; Acid Yellow 3, Yellow 10), 4-((4-Amino-3-sulfophenyl)azo)benzolsulfonsäure-dinatriumsalz (C.I. 13,015, Acid Yellow 9), 5-Hydroxy-1-(4-sulfophenyl)-4-[(4-sulfophenyl)azo]pyrazol-3-carbonsäure-trinatriumsalz (C.I. 19,140; Food Yellow No. 4; Acid Yellow 23), 3-[(4-Phenylamino)phenyl]azobenzolsulfonsäure-natriumsalz (C.I. 13,065; Ki406; Acid Yellow 36), 9-(2-Carboxyphenyl)-6-hydroxy-3H-xanthen-3-on (C.I. 45,350; Acid Yellow 73; D&C Yellow No. 8), 5-[(2,4-Dinitrophenyl)amino]-2-phenylaminobenzolsulfonsäure-natriumsalz (C.I. 10,385; Acid Orange 3), 4-[(2,4-Dihydroxyphenyl)azo]benzolsulfonsäure-natriumsalz (C.I. 14,270; Acid Orange 6), 4-[(2-Hydroxynaphth-1-yl)azo]benzolsulfonsäure-natriumsalz (C.I. 15,510; Acid Orange 7), 4-[(2,4-Dihydroxy-3-[(2,4-dimethylphenyl)azo]phenyl)azo]benzolsulfonsäure-natriumsalz (C.I. 20,170; Acid Orange 24).

Als kationische direktziehende Farbstoffe eignen sich insbesondere 3-[(4-Amino-2,5-dimethoxyphenyl)azo]-N,N,N-trimethylbenzolaminiumchlorid (C.I. 12,605, Basic Orange 69), Di[4-(dimethyl-amino)phenyl]iminomethan-hydrochlorid (C.I. 41,000; Basic Yellow 2), 2-[2-((2,4-Dimethoxyphenyl)amino)ethenyl]-1,3,3-trimethyl-3H-indol-1-ium-chlorid (C.I. 48,055; Basic Yellow 11), 3-Methyl-1-phenyl-4-[(3-(trimethylammonio)phenyl)azo]-pyrazol-5-on-chlorid (C.I. 12,719; Basic Yellow 57). Bevorzugte gelbe oder orange, kationische direktziehende Farbstoffe sind Basic Yellow 87 und Basic Orange 31.

Besonders bevorzugte gelbe oder orange, nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Yellow 13, HC Orange 1 und Disperse Orange 3, insbesondere HC Yellow 13.

Ein zweiter Gegenstand der vorliegenden Anmeldung ist ein Mittel zum Bleichen keratinischer Fasern gemäß Anspruch 3. Es hat sich erfindungsgemäß als besonders günstig herausgestellt, die Bleichmittel zunächst getrennt nach Oxidationsmittelzubereitung (B) und Zubereitung (A), enthaltend die für den Mattierungseffekt verantwortlichen Farbstoffe, bereitzustellen, um potentiellen Instabilitäten vorzubeugen.
Die erfindungsgemäßen Mittel zur Blondierung keratinischer Fasern enthalten mindestens eine Oxidationsmittelzubereitung (B). Diese enthält mindestens ein Oxidationsmittel, ausgewählt aus Wasserstoffperoxid und dessen Anlagerungsverbindungen an feste Träger.
Als erfindungsgemäßes Oxidationsmittel wird bevorzugt Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidinon ·n H₂O₂ (n ist eine positive ganze Zahl größer 0), Harnstoffperoxid und Melaminperoxid, eingesetzt. Erfindungsgemäß ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 3 Gew.-%ige bis 12 Gew.-%ige Lösungen in Wasser verwendet.
Die erfindungsgemäßen Mittel enthalten mit besonderem Vorzug Wasserstoffperoxid. Hier sind erfindungsgemäße Mittel zur Blondierung keratinischer Fasern besonders bevorzugt, die 0,5 bis 18 Gew.-%, vorzugsweise 1 bis 15 Gew.-%, besonders bevorzugt 2,5 bis 12 Gew.-% und insbesondere 3 bis 9 Gew.-% Wasserstoffperoxid (berechnet als 100%iges H₂O₂) enthalten.

Erfindungsgemäße Oxidationsmittelzubereitungen (B) sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 98 Gew.-%, vorzugsweise 60 bis 97,5 Gew.-%, besonders bevorzugt 70 bis 97 Gew.-%, weiter bevorzugt 75 bis 96 Gew.-% und insbesondere 80 bis 95 Gew.-% Wasser enthält.

Erfindungsgemäß kann aber das Oxidationsmittelzubereitung auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen. Geeignete Enzyme sind z. B. Peroxidasen, die die Wirkung geringer Mengen an Wasserstoffperoxid deutlich verstärken können. Ein Einsatz bestimmter Metallionen oder - komplexe kann ebenfalls bevorzugt sein. Geeignete Metallionen sind beispielsweise Zn²⁺, Cu²⁺, Fe²⁺, Fe³⁺, Mn²⁺, M⁴⁺, Li⁺, Mg²⁺, Ca²⁺, Ce⁴⁺, V³⁺, Co²⁺, Ru³⁺ und Al³⁺.

Die gebrauchsfertigen Blondiermittel aus Zubereitung (A) und Oxidationsmittelzubereitung (B) sollten bevorzugt einen pH-Wert im Bereich von 6 bis 12 aufweisen. Besonders bevorzugt ist die Anwendung im alkalischen Milieu. Die Anwendungstemperaturen können in einem Bereich zwischen 15 und 40 °C liegen. Nach einer Einwirkungszeit von 2 bis 60, bevorzugt 5 bis 45 Minuten wird das Blondiermittel durch Ausspülen von dem Haar entfernt. Das Nachwaschen mit einem Shampoo entfällt, wenn ein stark tensidhaltiger Träger verwendet wurde.

Weiterhin hat es sich als vorteilhaft erweisen, wenn die Oxidationsmittelzubereitungen mindestens einen Stabilisator oder Komplexbildner enthalten. Besonders bevorzugte Stabilisatoren sind Phenacetin, Alkalibenzoate (Natriumbenzoat) und Salicylsäure. Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Die Oxidationsmittelzubereitung (B) enthält neben dem eigentlichen Oxidationsmittel und gegebenenfalls Komplexbildner weitere Hilfs- und Zusatzstoffe. So hat es sich erfindungsgemäß als bevorzugt erwiesen, wenn die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Gemäß einer ersten bevorzugten Ausführungsform handelt es sich bei dem Verdickungsmittel um ein anionisches, synthetisches Polymer. Bevorzugte anionische Homopolymere sind beispielsweise unter dem Warenzeichnen Carbopol® im Handel erhältlich. Ebenfalls bevorzugt ist das Homopolymer der 2-Acrylamido-2-methylpropansulfonsäure, das beispielsweise unter der Bezeichnung Rheothik®11-80 im Handel erhältlich ist. Bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein bevorzugtes Handelsprodukt ist beispielsweise Aculyn® 33 der Firma Rohm & Haas. Weiterhin bevorzugte Copolymere werden von der Firma Rohm & Haas unter der Handelsbezeichnung Aculyn® 22 sowie von der Firma National Starch unter den Handelsbezeichnungen Structure® 2001 und Structure® 3001 sowie Carbopol® ETD 2020, Carbopol® Ultrez 20 und Pemulen TR-1 der Firma Lubrizol vertrieben. Bevorzugte anionische Copolymere sind weiterhin Acrylsäure-Acrylamid-Copolymere, wie in den Handelsprodukten Sepigel®305 und Simulgel® 600 der Firma SEPPIC enthalten. Weitere bevorzugte anionische Copolymere werden unter dem Handelsnamen Aristoflex® AVC bzw. Aristoflex® HMB von der Firma Clariant vertrieben. Auch Polymere aus Maleinsäureanhydrid und Methylvinylether, wie das unter der Bezeichnung Stabileze® QM im Handel erhältliche Copolymer, sind bevorzugte Verdickungsmittel. Bevorzugt kann das erfindungsgemäße Mittel zusätzlich mindestens ein anionisches Acrylsäure- und/oder Methacrylsäure-Polymerisat oder -Copolymerisat, wie Latekoll® D (BASF).

Gemäß einer zweiten Ausführungsform handelt es sich bei dem Verdickungsmittel um einen kationisches synthetisches Polymer. Ein besonders geeignetes Homopolymer ist das Poly(methacryloxyethyltrimethylammoniumChlorid) mit der INCI-Bezeichnung Polyquaternium-37. Dessen Polymerdispersionen sind unter den Bezeichnungen Salcare® SC 92, Salcare® SC 95 und Salcare® SC 96 im Handel erhältlich.

In einer dritten bevorzugten Ausführungsform werden natürlich vorkommende Verdickungsmittel eingesetzt. Nichtmodifizierte Guargums werden beispielsweise unter der Handelsbezeichnung Jaguar® C (Fa. Rhone Poulenc) vertrieben. Modifizierte Guargums sind unter den Bezeichnungen Jaguar® HP8, Jaguar® HP60, Jaguar® HP120, Jaguar® DC 293 und Jaguar® HP105 (Fa. Rhone Poulenc) im Handel erhältlich. Gemäß dieser Ausführungsform bevorzugt sind weiterhin Biosaccharidgums mikrobiellen Ursprungs, wie die Skleroglucangums oder Xanthangums, Gums aus pflanzlichen Exsudaten, wie beispielsweise Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen (Cellosize®; Fa. Amerchol; Natrosol®; Fa. Hercules sowie Blanose®; Fa. Aqualon, Aquasorb®, Ambergurri®; Fa. Hercules; Cellgon®; Fa. Montello). Bevorzugt sind weiterhin Stärke und deren Derivate, beispielsweise aus Kartoffeln, Maniok, Bataten, Maranta, Mais, Getreide, Reis, Hülsenfrüchten, Bananen oder dem Mark bestimmter Palmensorten gewonnen werden (Bsp. Amaze®; Fa. National Starch).

Auch nichtionische, vollsynthetische Polymere, wie beispielsweise Polyvinylalkohol oder Polyvinylpyrrolidinon, sind als erfindungsgemäße Verdickungsmittel einsetzbar (Luviskol®; Fa. BASF).

Als anorganische Verdickungsmittel haben sich Schichtsilikate als besonders geeignet im Sinne der vorliegenden Erfindung erwiesen (Optigel®; Fa. Süd Chemie).
Erfindungsgemäß bevorzugte Blondiermittel für keratinische Fasern sind dadurch gekennzeichnet, dass sie einen alkalischen pH-Wert besitzen. Eine weitere bevorzugte Ausführungsform der vorliegenden Erfindung besteht darin, dass das anwendungsbereite Mittel einen pH-Wert zwischen 7,0 und 12,0, bevorzugt zwischen 8,0 und 11,0 besitzt. Bei den pH-Werten im Sinne der vorliegenden Erfindung handelt es sich um pH-Werte, die bei einer Temperatur von 22 °C gemessen wurden. Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Ein insbesondere bevorzugtes Alkanolamin ist Monoethanolamin. Die erfindungsgemäßen Zusammensetzungen enthalten die Alkalisierungsmittel bevorzugt in Mengen von 0,2 bis 25 Gew.-%, insbesondere 0,5 bis 10 Gew.-%.
Eine besonders bevorzugte Darreichungsform des Mittels des zweiten Erfindungsgegenstands ist daher eine Verpackungseinheit (Kit-of-Parts), welche in getrennt voneinander konfektionierten Containern mindestens eine Oxidationsmittelzubereitung (B) und mindestens eine Zubereitung (A) enthält, wobei die Zubereitung (A) ein Mittel gemäß Anspruch 1 ist. Weiterhin kann es erfindungsgemäß besonders vorteilhaft sein, wenn das genannte Kit-of-Parts mindestens ein weiteres Haarbehandlungsmittel in einem getrennten Container enthält, insbesondere ein Konditioniermittel.
Darüber hinaus kann die Verpackungseinheit Applikationshilfen, wie Kämme, Bürsten oder Pinsel, persönliche Schutzkleidung, insbesondere Ein-Weg-Handschuhe, sowie gegebenenfalls eine Gebrauchsanleitung umfassen. Hinsichtlich der bevorzugten Ausführungsführungsformen der Zubereitung (A) und der Oxidationsmittelzubereitung (B) gelten die obigen Ausführungen des ersten und zweiten Erfindungsgegenstands.

Wird eine starke Aufhellung gewünscht, so ist es erfindungsgemäß bevorzugt, wenn zusätzlich eine Blondierzubereitung (C), enthaltend mindestens einen Bleichkraftverstärker, der Mischung aus Oxidationsmittelzubereitung (B) und der Zubereitung (A) beigemischt wird. Es kann dabei unerheblich sein, ob zunächst eine Mischung (A) und (B) hergestellt wird, und anschließend die Blondierzubereitung (C) zugemischt wird, oder ob eine davon verschiedene Reihenfolge der Vermischung der einzelnen Komponenten genutzt wird. Es ist bevorzugt, die einzelnen Zubereitungen in möglichst naher zeitlicher Abfolge zu vermischen und das anwendungsbereite Mittel vorzugsweise zeitnah auf die keratinischen Fasern zu applizieren.
Eine weitere Ausführungsform der vorliegenden Anmeldung ist daher ein Mittel zum Bleichen keratinischer Fasern, dadurch gekennzeichnet, dass es durch Vermischen von mindestens einer Oxidationsmittelszubereitung (B), enthaltend mindestens ein Oxidationsmittel, ausgewählt aus Wasserstoffperoxid und dessen Anlagerungsverbindungen an feste Träger, mindestens einer Blondierzubereitung (C), enthaltend mindestens einen Bleichkraftverstärker, und mindestens einer Zubereitung (A) hergestellt wird, wobei die Zubereitung (A) ein Mittel gemäß Anspruch 1 ist. Es ist erfindungsgemäß besonders vorteilhaft, wenn im anwendungsbereiten Mittel zu Bleichen keratinischer Fasern die Gesamtmenge an direktziehenden Farbstoffen 0,0001 bis 0,2 Gew.-%, bezogen auf das Gesamtgewicht des anwendungsbereiten Mittels, beträgt.
Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in der Regel eine Kombination aus Wasserstoffperoxid und mindestens eines Bleichkraftverstärkers eingesetzt, woraus eine Steigerung des Aufhellvermögens der Mittel resultiert.
Als Bleichkraftverstärker können im Rahmen dieser Erfindung Peroxoverbindungen, weiterhin Verbindungen, die unter Perhydrolysebedingungen aliphatische Peroxocarbonsäuren und/oder substituierte Perbenzoesäure ergeben, Kohlensäurederivate, Alkylcarbonate, -carbamate, Silylcarbonate, -carbamate und stickstoffhaltige, gegebenenfalls kationische Heterocyclen, insbesondere Acylpyridinium und Dihydroisochinolinium-Salze, eingesetzt werden.
Vorzugsweise ist der Bleichkraftverstärker ausgewählt aus Ammoniumpersulfat, Alkalimetallpersulfaten, Ammoniumperoxomonosulfat, Alkalimetallhydrogenperoxomonosulfaten, Alkalimetallperoxodiphosphaten und Erdalkalimetallperoxiden. Besonders bevorzugte Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid. Erfindungsgemäß besonders bevorzugt sind Mittel, die in der Blondierzubereitung (C) als Bleichkraftverstärker mindestens ein anorganisches Salz, ausgewählt aus Persulfaten und/oder Peroxodisulfaten, enthalten. Weiterhin hat es sich bei den Arbeiten zur vorliegenden Erfindung als bevorzugt erwiesen, wenn die erfindungsgemäßen Mittel mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat. Die Peroxoverbindungen sind in einer Menge von 0,1 bis 25 Gew.-%, insbesondere in einer Menge von 0,5 bis 15 Gew.-%, bezogen auf das Gesamtgewicht des anwendungsbereiten Mittels, enthalten.

Der Einsatz von Persulfatsalzen bzw. Peroxodisulfatsalzen erfolgt in der Regel in Form eines gegebenenfalls entstaubten Pulvers, Paste oder eines in Form gepressten Formkörpers.

Die erfindungsgemäßen wasserfreien Zusammensetzungen anstelle und/oder zusätzlich zu den festen Peroxoverbindungen einen weiteren Bleichkraftverstärker enthalten. Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. i-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran. Als Bleichverstärker vom Typ der Kohlensäurederivate können erfindungsgemäß bevorzugt Carbonatsalze bzw. Hydrogencarbonatsalze eingesetzt werden. Diese sind bevorzugt ausgewählt aus der Gruppe der Ammonium-, Alkalimetall- (insbesondere Natrium- und Kalium-) sowie Erdalkalimetall- (insbesondere Magnesium- und Calcium-), -carbonatsalze bzw. - hydrogencarbonatsalze. Besonders bevorzugte Carbonat- bzw. Hydrogencarbonatsalze sind Ammoniumhydrogencarbonat, Ammoniumcarbonat, Natriumhydrogencarbonat, Natriumcarbonat, Kaliumhydrogencarbonat, Kaliumcarbonat, Magnesiumcarbonat und Calciumcarbonat. Diese bevorzugten Salze können allein oder in deren Mischungen von mindestens zwei Vertretern als Bleichverstärker verwendet werden. Weiterhin erfindungsgemäß einsetzbare Bleichkraftverstärker können aus stickstoffhaltigen, gegebenenfalls kationischen Heterocyclen. Als Beispiel eines stickstoffhaltigen heterocyclischen Bleichkraftverstärkers ist insbesondere Imidazol zu nennen.

Besonders bevorzugte stickstoffhaltige, heterocyclische Bleichkraftverstärker sind die quaternierten Kationen von Pyridinen und 3,4-Dihydroisochinolinen. Bevorzugte Verbindungen sind dabei 4-Acetyl-1-methylpyridinium-p-toluolsulfonat, 4-Acetyl-1-methylpyridiniumbenzolsulfonat, 4-Acetyl-1-methylpyridiniumbromid, 4-Acetyl-1-methylpyridiniumhydrogensulfat, 4-Acetyl-1-allylpyridinium-p-toluolsulfonat, 4-Acetyl-1-allylpyridiniumbenzolsulfonat, 4-Acetyl-1-allylpyridiniumbromid, 4-Acetyl-1-allylpyridiniumhydrogensulfat, 4-Acetyl-1-(2-hydroxyethyl)pyridinium-p-toluolsulfonat, 4-Acetyl-1-(2-hydroxyethyl)pyridiniumbenzolsulfonat, 4-Acetyl-1-(2-hydroxyethyl)pyridiniumbromid, 4-Acetyl-1-(2-hydroxyethyl)pyridiniumhydrogensulfat, 4-Acetyl-1-(2-oxopropyl)pyridinium-p-toluolsulfonat, 4-Acetyl-1-(2-oxopropyl)pyridiniumbenzolsulfonat, 4-Acetyl-1-(2-oxopropyl)pyridiniumbromid, 4-Acetyl-1-(2-oxopropyl)pyridiniumhydrogensulfat, 4-Acetyl-1-ethylpyridinium-p-toluolsulfonat, 4-Acetyl-1-ethylpyridiniumbenzolsulfonat, 4-Acetyl-1-ethylpyridiniumbromid, 4-Acetyl-1-ethylpyridiniumhydrogensulfat, 4-Acetyl-1-(2-methyl-prop-2-enyl)pyridinium-p-toluolsulfonat, 4-Acetyl-1-(2-methyl-prop-2-enyl)pyridiniumbenzolsulfonat, 4-Acetyl-1-(2-methyl-prop-2-enyl)pyridinium-bromid, 4-Acetyl-1-(2-methyl-prop-2-enyl)pyridiniumhydrogensulfat, 4-Acetyl-1-benzylpyridinium-p-toluolsulfonat, 4-Acetyl-1-benzylpyridiniumbenzolsulfonat, 4-Acetyl-1-benzylpyridiniumbromid, 4-Acetyl-1-benzylpyridiniumhydrogensulfat, 4-Acetyl-1-(2-methoxyethyl)pyridinium-p-toluolsulfonat, 4-Acetyl-1-(2-methoxyethyl)pyridiniumbenzolsulfonat, 4-Acetyl-1-(2-methoxyethyl)pyridiniumbromid oder 4-Acetyl-1-(2-methoxyethyl)pyridiniumhydrogensulfat sowie N-Methyl-3,4-dihydroisochinolinium-p-toluolsulfonat, N-Methyl-3,4-dihydroisochinoliniumbenzolsulfonat, N-Methyl-3,4-dihydroisochinoliniumhydrogensulfat, N-Allyl-3,4-dihydroisochinolinium-p-toluolsulfonat, N-Allyl-3,4-dihydroisochinoliniumbenzolsulfonat, N-Allyl-3,4-dihydroisochinoliniumbromid, N-Allyl-3,4-dihydro-isochinoliniumacetat, 3,4-Dihydro-2-(3-hydroxypropyl)isochinolinium-p-toluolsulfonat, 3,4-Dihydro-2-(3-hydroxypropyl)isochinoliniumbenzolsulfonat, 3,4-Dihydro-2-(3-hydroxypropyl)isochinolinium-bromid, 3,4-Dihydro-2-(3-hydroxypropyl)isochinoliniumacetat, 3,4-Dihydro-2-(2-hydroxyethyl)iso-chinolinium-p-toluolsulfonat, 3,4-Dihydro-2-(2-hydroxyethyl)isochinoliniumbenzolsulfonat, 3,4-Dihydro-2-(2-hydroxyethyl)isochinoliniumbromid oder 3,4-Dihydro-2-(2-hydroxyethyl)isochinolinium-acetat.

Die neben oder anstelle von Peroxoverbindungen eingesetzten Bleichkraftverstärker sind in den erfindungsgemäßen, kosmetischen Mitteln bevorzugt in Mengen von 0,5 bis 30 Gew.-%, insbesondere in Mengen von 2 bis 20 Gew.-%, jeweils bezogen auf das Gesamtgewicht des anwendungsbereiten Mittels, enthalten.

Zur weiteren Steigerung der Aufhellleistung können der erfindungsgemäßen Zusammensetzung zusätzlich als Bleichverstärker mindestens eine gegebenenfalls hydratisierte SiO₂-Verbindung zugesetzt. Obwohl bereits geringe Mengen der gegebenenfalls hydratisierten SiO₂-Verbindungen die Aufhellleistung erhöhen, kann es erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder. Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt. Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt. Diese SiO₂-Verbindungen können teilweise in wässriger Lösung vorliegen. Erfindungsgemäß ganz besonders bevorzugt sind Wassergläser, die aus einem Silikat der Formel (SIO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt. Insbesondere Metasilicate, die sich gemäß vorstehender Formel durch das Verhältnis zwischen n und der Summe aus m und p von ≤ 1 auszeichnen und sich als kettenförmige polymere Strukturen des Anions [SiO₃]²⁻ auffassen lassen, können bevorzugt eingesetzt werden. Natriummetasilicat der Formel [NaSiO₃]ₓ, ist dabei besonders bevorzugt. Erfindungsgemäß besonders bevorzugte Wassergläser werden unter anderem von der Firma Henkel unter den Bezeichnungen Ferrosil® 119, Natronwasserglas 40/42, Portil® A, Portil® AW und Portil® W und von der Firma Akzo unter der Bezeichnung Britesil® C20 vertrieben.

Obwohl prinzipiell keine Einschränkungen hinsichtlich der Formulierung der Blondierzubereitung (C) bestehen, hat es sich erfindungsgemäß als bevorzugt erwiesen, wenn die Zubereitung (C) wasserfrei formuliert ist. Wasserfrei im Sinne der vorliegenden Erfindung bedeutet einen Wassergehalt bezogen auf die Zubereitung (C) von weniger als 5 Gew.-%, insbesondere von weniger als 2 Gew.-%. Blondierzubereitungen, die weniger als 0,1 Gew.-% Wasser, enthalten können erfindungsgemäß ganz besonders bevorzugt sein.

Die Zubereitung (C) ist vorzugsweise als Pulver oder als wasserfreie Paste formuliert. Im Falle der Formulierung als wasserfreie Paste hat es sich als besonders bevorzugt erwiesen, wenn die Zubereitung (C) mindestens einen nicht hydroxylierten Fettsäureester mit einem Schmelzpunkt von höchstens 50°C, insbesondere von höchstens 30°C, und/oder mindestens eine C₁₀-C₃₀-Fettsäure mit mindestens einer zusätzlichen Hydroxygruppe und/oder ein Derivat davon, enthält.

Ester von nicht hydroxylierten C₆-C₃₀-Alkylmonocarbonäuren mit C₂-C₃₀-Monoalkoholen eignen sich erfindungsgemäß bevorzugt als Fettsäureester. Bevorzugt sind die Monoester der Fettsäuren mit Monoalkoholen mit 2 bis 24 C-Atomen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Kokosfettalkohol-caprinat/- caprylat (Cetiol® LC), n-Butylstearat, Oleylerucat (Cetiol® J 600), Isopropylpalmitat (Rilanit® IPP), Oleyloleat (Cetiol®), Laurinsäurehexylester (Cetiol® A), Myristylmyristat (Cetiol® MM), Cetearylisononanoat (Cetiol® SN), Ölsäuredecylester (Cetiol® V).
Hinsichtlich der bevorzugten Ausführungsführungsformen der Zubereitung (A) und der Oxidationsmittelzubereitung (B) gelten die obigen Ausführungen des ersten und zweiten Erfindungsgegenstands.
Eine besonders bevorzugte Darreichungsform des Mittels ist weiterhin eine Verpackungseinheit (Kit-of-Parts), welche in getrennt voneinander konfektionierten Containern mindestens eine Oxidationsmittelzubereitung (B), enthaltend mindestens ein Oxidationsmittel, ausgewählt aus Wasserstoffperoxid und dessen Anlagerungsverbindungen an feste Träger, mindestens einer Blondierzubereitung (C), enthaltend mindestens einen Bleichkraftverstärker, und mindestens eine Zubereitung (A) enthält, wobei die Zubereitung (A) ein Mittel gemäß Anspruch 1 ist Hinsichtlich der bevorzugten Ausführungsführungsformen der Zubereitung (A) und der Oxidationsmittelzubereitung (B) und der Blondierzubereitung (C) gelten die obigen Ausführungen.
Die Mischung der Zubereitungen (A) und (B) bzw. gegebenenfalls der Zubereitungen (A), (B) und (C) vor der Anwendung führt zu einer erfindungsgemäßen Anwendungsmischung.
Vorzugsweise sind die Oxidationsmittelzubereitung (B) und/oder die die mattierenden Farbstoffe enthaltende Zubereitung (A) als fließfähigen Zubereitungen konfektioniert. Vorzugsweise wird den fließfähigen Zubereitungen (A) und/oder (B) weiterhin ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind.
Als anionische Tenside eignen sich in erfindungsgemäßen Zubereitungen alle für die Verwendung am menschlichen Körper geeigneten anionischen oberflächenaktiven Stoffe. Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül. Geeignete zwitterionische Tenside sind die sogenannten Betaine wie die N-Alkyl-N,N-dimethylammonium-glycinate, N-Acyl-aminopropyl-N,N-dimethylammoniumglycinate und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist unter der INCI-Bezeichnung Cocamidopropyl Betaine bekannt. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das C₁₂-C₁₈-Acylsarcosin. Weiterhin hat es sich als vorteilhaft erwiesen, wenn die erfindungsgemäßen Aufhellmittel weitere, nichtionogene grenzflächenaktive Stoffe, die sie nicht unter die oben beschriebenen nichtionischen Komponenten mit einem HLB-Wert von 8,0 oder kleiner fallen, enthalten. Als nichtionische Tenside eignen sich insbesondere C₈-C₂₂-Alkylmono- und oligoglycoside und deren ethoxylierte Analoga. Insbesondere die nichtethoxylierten Verbindungen haben sich als besonders geeignet erwiesen. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten. Die anionischen, nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar. Die Alkylamidoamine werden üblicherweise durch Amidierung natürlicher oder synthetischer Fettsäuren und Fettsäureschnitte mit Dialkylaminoaminen hergestellt (Bsp.Tegoamid® S 18: Stearamidopropyl-dimethylamin). Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis-(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind bevorzugt.
In einer bevorzugten Ausführungsform können nicht-ionische, zwitterionische und/oder amphotere Tenside sowie deren Mischungen bevorzugt sein. Bevorzugt können die erfindungsgemäßen Zusammensetzungen mindestens einen nichtionogenen Emulgator bzw. Tensid mit einem HLB-Wert von 8 bis 18 enthalten. Nichtionogene Emulgatoren bzw. Tenside mit einem HLB-Wert von 10-15 können erfindungsgemäß besonders bevorzugt sein.
Die erfindungsgemäßen Mittel können weiterhin alle für solche Zubereitungen bekannten Wirk-, Zusatz- und Hilfsstoffe enthalten. Weitere Wirk-, Hilfs- und Zusatzstoffe sind beispielsweise strukturverbessernde Wirkstoffe (Pflanzenextrakte, Proteinhydrolysate, Panthenol, Milchsäure, Biotin, Niacinamid, Mono-, Di- und Oligosaccharide, Serin, Lysin); Silikonöle; Phospholipide; pflanzliche Öle; nichtionische Polymere; zwitterionische und amphotere Polymere; anionische Polymere; kationische Polymere (Celquat® H 100, Celquat® L 200; Polymer JR®400; Merquat®100; Merquat®550; Merquat® 280; Gafquat®734 und Gafquat®755; Luviquat®; quaternierter Polyvinylalkohol; Polyquaternium-1, Polyquaternium 2, Polyquaternium-10; Polyquaternium 17, Polyquaternium 18, Polyquaternium-22 und Polyquaternium 27); Parfümöle; Cyclodextrine; Entschäumer wie Silikone; Farbstoffe zum Anfärben des Mittels; Antischuppenwirkstoffe; Lichtschutzmittel, insbesondere derivatisierte Benzophenone, Zimtsäure-Derivate und Triazine; Cholesterin; weitere Fette und Wachse wie Fettalkohole, Bienenwachs, Montanwachs und Paraffine; Fettsäurealkanolamide; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel; Pigmente; Treibmittel wie Propan-Butan-Gemische, N₂O Dimethylether, CO₂ und Luft sowie Antioxidantien. Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.
Ein weiterer Gegenstand der vorliegenden Erfindung ist ein Verfahren zur Blondierung keratinischer Fasern, insbesondere menschlicher Haare, wobei ein Mittel durch Vermischen von mindestens einer Oxidationsmittelszubereitung (B), enthaltend mindestens ein Oxidationsmittel, ausgewählt aus Wasserstoffperoxid und dessen Anlagerungsverbindungen an feste Träger, mindestens einer Blondierzubereitung (C), enthaltend mindestens einen Bleichkraftverstärker, und mindestens einer Zubereitung (A) hergestellt wird, wobei die Zubereitung (A) ein Mittel gemäß Anspruch 1 ist, das vermischte Mittel für einen Zeitraum von 2 bis 60 Minuten, bevorzugt 5 bis 45 Minuten im Haar belassen wird, und das Haar anschließend mit Wasser und/oder einem handelsüblichen Shampoo gespült wird. Im Rahmen dieses Gegenstandes der vorliegenden Erfindung gelten die oben getroffenen Aussagen analog.
So hat es sich beispielsweise als besonders vorteilhaft erwiesen, wenn die Zubereitungen (A) und (B) wässrig formuliert sind, wohingegen die Blondierzubereitung (C) vorzugsweise wasserfrei formuliert ist. Weiterhin ist es erfindungsgemäß bevorzugt, wenn die Zubereitung (C) pulverförmig ist.

Ein weiterer Gegenstand der vorliegenden Anmeldung ist schließlich die Verwendung eines Mittels gemäß einem der vorgenannten Erfindungsgegenstände zur Mattierung während der Aufhellung und/oder Blondierung keratinischer Fasern, insbesondere menschlicher Haare. Im Rahmen dieses Gegenstandes der vorliegenden Erfindung gelten die oben getroffenen Aussagen analog.
Die folgenden Beispiele sollen den Gegenstand der vorliegenden Erfindung näher erläutern ohne diesen in irgendeiner Weise zu beschränken.

### Beispiele

Die Mengenangaben in den Beispielen verstehen sich, soweit nichts anderes vermerkt ist, in Gewichtsprozent.
Es wurden die folgenden Zubereitungen hergestellt:

### 1 Mattierungsmittel

| **Rohstoff** | **Menge [Gew.-%]** |
|---|---|
| Dow Corning 193 Fluid | 0,2 |
| Ammoniak 25 % | 2,0 |
| Synthalen K | 0,04 |
| Cetiol V | 2,30 |
| Lanette N | 14,00 |
| Cetearyl Alcohol | 3,90 |
| Cutina GMS SE | 6,00 |
| Phospholipid EFA | 0,10 |
| Na₄ EDTA Pulver 87% | 0,80 |
| Monoethanolamin | 5,00 |
| Farbstoffrezeptur* | 0,053 |
| L-Serin | 0,50 |
| Mirapol A-15 | 0,20 |
| Parfum | qs |
| Wasser | ad 100 |

| | |
|---|---|
| *bestehend aus Tetrabromphenolblau (75,5 Gew.-%), Acid Red 92 (18,8 Gew.-%) und HC Yellow No. 13 (5,7 Gew.-%), jeweils bezogen auf das Gesamtgewicht der Farbstoffrezeptur. | |

### 2 Oxidationsmittelzubreitung

| **Rohstoff** | **Menge [Gew.-%]** |
|---|---|
| NaBenzoat | 0,04 |
| Dipicolinsäure | 0,10 |
| Dinatriumpyrophosphat | 0,10 |
| Kaliumhydroxid 50% | 0,19 |
| 1,2-Propandiol | 1,50 |
| HEDP 60% | 0,25 |
| Paraffinum Liquidum | 0,30 |
| Genamin STAC | 0,39 |
| Cetearyl Alcohol | 3,40 |
| Eumulgin B 2 | 1,00 |
| Wasserstoffperoxid 50 % | 18,20 |
| Wasser | add 100 |

### 3 Bleichkraftverstärkerzubereitung

| **Rohstoff** | **Menge [Gew.-%]** |
|---|---|
| Natriummetasilikat | 4,6 |
| Portil N | 38,0 |
| Rohagit S hv | 0,5 |
| Cekol 50000 | 2,5 |
| Magnesiumcarbonat | 14,2 |
| Na₂EDTA | 1,0 |
| Gluadin AGP | 0,1 |
| Kieselsäure, pyrogen | 0,5 |
| Kaliumpersulfat | 20,4 |
| Natriumpersulfat | 10,2 |
| Paraffinum Liquidum | 8,0 |

### 4 Blondierung

Unmittelbar vor der Anwendung auf den Fasern wurden 15g Mattierungsmittel mit 70g Oxidationsmittelzubereitung und 35g Bleichkraftverstärkerzubereitung vermischt. Die resultierende Anwendungszubereitung wurde auf hellbraune bzw. mittelbraune Strähnen in einem Flottenverhältnis von 4 zu 1 aufgetragen und 45 Minuten bei 35 °C auf den Strähnen belassen. Anschließend wurden die Strähnen mit Wasser gründlich gespült und mit Hilfe eines Föns getrocknet.

Es wurde eine hervorragende Aufhellung mit guten Farbausgleich mit leichter cendre-Note ohne unerwünschte Orange- bzw. Rotverschiebung erhalten.

### 5 Verzeichnis der eingesetzten Handelsprodukte

| | |
|---|---|
| Dow Corning® 193 Fluid | INCI-Bezeichnung: PEG-12 Dimethicone (Dow Corning) |
| Synthalen® K | INCI-Bezeichnung: Carbomer (3V Sigma) |
| Cetiol® V | INCI-Bezeichnung: Decyl Oleate (Cognis) |
| Lanette® N | INCI-Bezeichnung: Sodium Cetearyl Sulfate, Cetearyl Alcohol (Cognis) |
| Cutina® GMS SE | INCI-Bezeichnung: Glycerin, Glyceryl Stearate, Potassium Stearate (Cognis) |
| Phospholipid EFA® | INCI-Bezeichnung: Linoleamidopropyl PG-Dimonium Chloride Phosphate (Uniqema) |
| Mirapol® A-15 | INCI-Bezeichnung: Polyquaternium-2 (Rhodia) |
| Genamin® STAC | INCI-Bezeichnung: Steartrimoniumchloride (Clariant) |
| Eumulgin® B 2 | INCI-Bezeichnung: Ceteareth-20 (Cognis) |
| Portil® N | INCI-Bezeichnung: Sodium Silicate (Cognis) |
| Rohagit® S hv | INCI-Bezeichnung: Acrylates Copolymer (Evonik) |
| Cekol® 50000 | INCI-Bezeichnung: Cellulose Gum (Kelco) |
| Gluadin® AGP | INCI-Bezeichnung: hydrolyzed wheat protein (Cognis) |

## Patentansprüche

1. Mittel, enthaltend in einem kosmetischen Träger mindestens einen nichtionischen Emulgator mit einem HLB-Wert von kleiner oder gleich 8,0, **dadurch gekennzeichnet, dass** es zusätzlich eine Kombination aus mindestens einem blauen direktziehenden Farbstoff und einem roten direktziehenden Farbstoff enthält, wobei das Gewichtsverhältnis zwischen der Summe aller blauen direktziehenden Farbstoffe und der Summe aller roten direktziehenden Farbstoffe einen Wert von größer oder gleich 1 besitzt,
**dadurch gekennzeichnet, dass** das Mittel als blauen direktziehenden Farbstoff eine Verbindung gemäß Formel (I) und/oder eines ihrer physiologisch verträglichen Salze 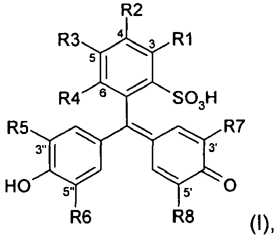 worin R1, R2, R3, R4, R5, R6, R7 und R8 für Brom stehen, enthält und **dadurch gekennzeichnet, dass** das Mittel als roten direktziehenden Farbstoff eine Verbindung gemäß Formel (II) und/oder eines ihrer physiologisch verträglichen Salze 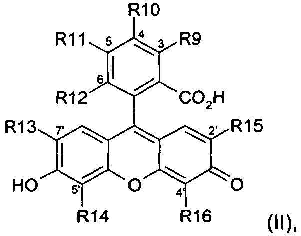 worin R9, R10, R11 und R12 für Chlor und R13, R14, R15 und R16 für Brom stehen,
enthält und dass es
als nichtionischen Emulgator mit einem HLB-Wert von kleiner oder gleich 8,0 mindestens einen Fettalkohol mit 12, 14, 16 oder 18 Kohlenstoffatomen in der Alkylkette enthält.

2. Mittel nach Anspruch 1, **dadurch gekennzeichnet, dass** das Gewichtsverhältnis zwischen der Summe aller blauen direktziehenden Farbstoffe und der Summe aller roten direktziehenden Farbstoffe einen Wert von 1 bis 100, bevorzugt von 1,5 bis 10 und besonders bevorzugt von 2 bis 4 besitzt.

3. Mittel zum Bleichen keratinischer Fasern, **dadurch gekennzeichnet, dass** es durch Vermischen von mindestens einer Oxidationsmittelszubereitung (B), enthaltend mindestens ein Oxidationsmittel, ausgewählt aus Wasserstoffperoxid und dessen Anlagerungsverbindungen an feste Träger, und mindestens einer Zubereitung (A) hergestellt wird, wobei die Zubereitung (A) in einem kosmetischen Träger mindestens eine nichtionische Komponente mit einem HLB-Wert von kleiner oder gleich 8,0 und eine Kombination aus mindestens einem blauen direktziehenden Farbstoff und einem roten direktziehenden Farbstoff enthält, wobei das Gewichtsverhältnis zwischen der Summe aller blauen direktziehenden Farbstoffe und der Summe aller roten direktziehenden Farbstoffe einen Wert von größer oder gleich 1 besitzt,
**dadurch gekennzeichnet, dass** die Zubereitung (A) als blauen direktziehenden Farbstoff eine Verbindung gemäß Formel (I) und/oder eines ihrer physiologisch verträglichen Salze 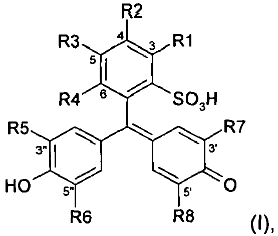 worin R1, R2, R3, R4, R5, R6, R7 und R8 für Brom stehen, enthält und **dadurch gekennzeichnet, dass** die Zubereitung (A) als roten direktziehenden Farbstoff eine Verbindung gemäß Formel (II) und/oder eines ihrer physiologisch verträglichen Salze 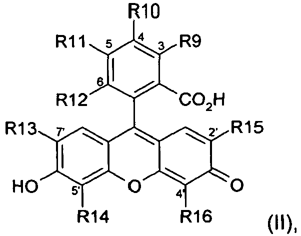 worin R9, R10, R11 und R12 für Chlor und R13, R14, R15 und R16 für Brom stehen,
enthält
und dass sie als nichtionischen Emulgator mit einem HLB-Wert von kleiner oder gleich 8,0 mindestens einen Fettalkohol mit 12, 14, 16 oder 18 Kohlenstoffatomen in der Alkylkette enthält.

4. Mittel nach Anspruch 3, **dadurch gekennzeichnet, dass** es durch Vermischen mit zusätzlich mindestens einer Blondierzubereitung (C), enthaltend mindestens einen Bleichkraftverstärker, hergestellt wird.

5. Verfahren, bei dem ein Mittel gemäß Anspruch 3 auf menschliches Haar aufgetragen wird, das Mittel für einen Zeitraum von 2 bis 60 Minuten, bevorzugt 5 bis 45 Minuten im Haar belassen wird, und das Haar anschließend mit Wasser und/oder einem handelsüblichen Shampoo gespült wird.

6. Verwendung eines Mittels gemäß Anspruch 3 zur Mattierung während der Aufhellung und/oder Blondierung keratinischer Fasern, insbesondere menschlicher Haare.

## Claims

1. An agent containing, in a cosmetic carrier, at least one non-ionic emulsifier having an HLB value of less than or equal to 8.0, **characterized in that** it additionally contains a combination of at least one blue direct dye and one red direct dye, the weight ratio between the sum of all blue direct dyes and the sum of all red direct dyes having a value of greater than or equal to 1, **characterized in that** the agent contains a compound according to formula (I) and/or one of the physiologically acceptable salts thereof as a blue direct dye, 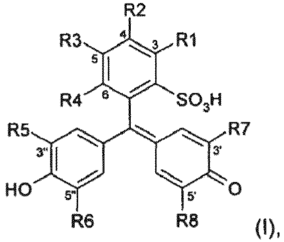 where R1, R2, R3, R4, R5, R6, R7 and R8 represent bromine,
and
**characterized in that** the agent contains a compound according to formula (II) and/or one of the physiologically acceptable salts thereof as a red direct dye, 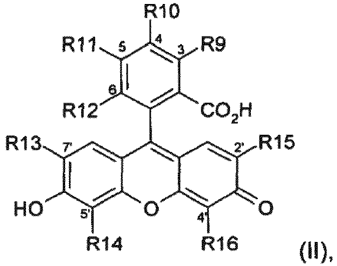 where R9, R10, R11 and R12 represent chlorine and R13, R14, R15 and R16 represent bromine, and **in that** it
contains at least one fatty alcohol having 12, 14, 16 or 18 carbon atoms in the alkyl chain as a non-ionic emulsifier having an HLB value of less than or equal to 8.0.

2. The agent according to claim 1, **characterized in that** the weight ratio between the sum of all blue direct dyes and the sum of all red direct dyes has a value of from 1 to 100, preferably from 1.5 to 10, and particularly preferably from 2 to 4.

3. An agent for bleaching keratin fibers, **characterized in that** it is prepared by mixing at least one oxidizing agent preparation (B), containing at least one oxidizing agent, selected from hydrogen peroxide and addition compounds thereof on solid carriers, and at least one preparation (A), the preparation (A) containing, in a cosmetic carrier, at least one non-ionic component having an HLB value of less than or equal to 8.0 and a combination of at least one blue direct dye and one red direct dye, the weight ratio between the sum of all blue direct dyes and the sum of all red direct dyes having a value of greater than or equal to 1,
**characterized in that** the preparation (A) contains a compound according to formula (I) and/or one of the physiologically acceptable salts thereof as a blue direct dye, 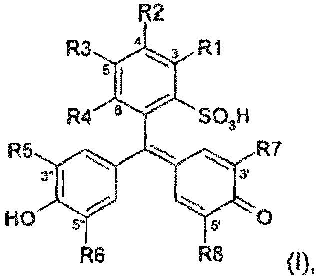 where R1, R2, R3, R4, R5, R6, R7 and R8 represent bromine,
and **characterized in that** the preparation (A) contains a compound according to formula (II) and/or one of the physiologically acceptable salts thereof as a red direct dye, 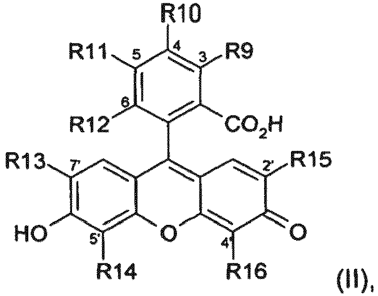 where R9, R10, R11 and R12 represent chlorine and R13, R14, R15 and R16 represent bromine, and **in that** it contains at least one fatty alcohol having 12, 14, 16 or 18 carbon atoms in the alkyl chain as a non-ionic emulsifier having an HLB value of less than or equal to 8.0.

4. The agent according to claim 3, **characterized in that** it is prepared by mixing with additionally at least one bleaching preparation (C) containing at least one bleach enhancer.

5. A method in which an agent according to claim 3 is applied to human hair, the agent is left in the hair for a period of from 2 to 60 minutes, preferably from 5 to 45 minutes, and the hair is subsequently rinsed with water and/or a commercially available shampoo.

6. The use of an agent according to claim 3 for matting during the lightening and/or bleaching of keratin fibers, in particular human hair.

## Revendications

1. Agent contenant, dans un support cosmétique, au moins un émulsifiant non ionique ayant une valeur HLB inférieure ou égale à 8,0, **caractérisé en ce qu'**il contient en outre une combinaison d'au moins un colorant direct bleu et un colorant direct rouge, le rapport en poids entre la somme de tous les colorants directs bleus et la somme de tous les colorants directs rouges ayant une valeur supérieure ou égale à 1,
**caractérisé en ce que** l'agent contient comme colorant direct bleu un composé de la formule (I) et/ou l'un de ses sels physiologiquement acceptables 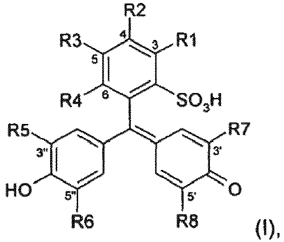 où R1, R2, R3, R4, R5, R6, R7 et R8 représentent un atome de brome et
**caractérisé en ce que** l'agent contient comme colorant direct rouge un composé de la formule (II) et/ou l'un de ses sels physiologiquement acceptables 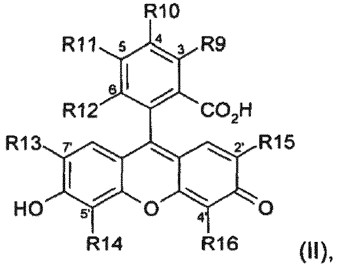 où R9, R10, R11 et R12 représentent des atomes de chlore et R13, R14, R15 et R16 représentent des atomes de brome,
et **en ce qu'**il contient comme émulsifiant non ionique ayant une valeur HLB inférieure ou égale à 8,0 au moins un alcool gras ayant 12, 14, 16 ou 18 atomes de carbone dans la chaîne alkyle.

2. Composition selon la revendication 1, **caractérisé en ce que** le rapport en poids entre la somme de tous les colorants directs bleus et la somme de tous les colorants directs rouges a une valeur de 1 à 100, de préférence de 1,5 à 10 et de manière particulièrement préférée de 2 à 4.

3. Agent de décoloration de fibres de kératine, **caractérisé en ce qu'**il est produit par mélange d'au moins une préparation d'agent oxydant (B), contenant au moins un agent oxydant choisi parmi le peroxyde d'hydrogène et ses composés d'addition à des supports solides, et d'au moins une composition (A), la préparation (A) contenant dans un support cosmétique au moins un composant non ionique ayant une valeur HLB inférieure ou égale à 8,0, et une combinaison d'au moins un colorant direct bleu et un colorant direct rouge, le rapport en poids entre la somme de tous les colorants directs bleus et la somme de tous les colorants directs rouges ayant une valeur supérieure ou égale à 1,
**caractérisé en ce que** la préparation (A) contient comme colorant direct bleu, un composé selon la formule (I) et/ou l'un de ses sels physiologiquement acceptables 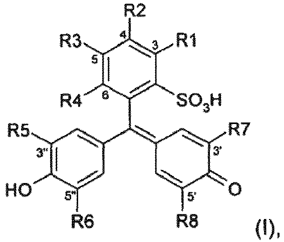 où R1, R2, R3, R4, R5, R6, R7 et R8 représentent un atome de brome,
et **caractérisé en ce que** la préparation (A) contient comme colorant direct rouge un composé selon la formule (II) et/ou l'un de ses sels physiologiquement acceptables 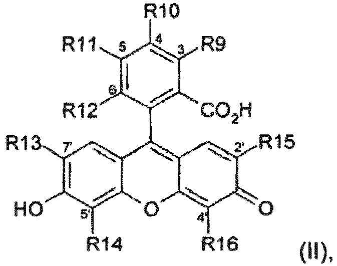 où R9, R10, R11 et R12 représentent des atomes de chlore et R13, R14, R15 et R16 représentent des atomes de brome,
et ce qu'elle contient comme émulsifiant non ionique ayant une valeur HLB inférieure ou égale à 8,0 au moins un alcool gras ayant 12, 14, 16 ou 18 atomes de carbone dans la chaîne alkyle.

4. Agent selon la revendication 3, **caractérisé en ce qu'**il est produit par mélange avec en outre au moins une préparation de blondissement (C) contenant au moins un amplificateur du pouvoir décolorant.

5. Procédé dans lequel un agent selon la revendication 3 est appliqué sur des cheveux humains, l'agent est laissé pendant une durée allant de 2 à 60 minutes, de préférence de 5 à 45 minutes sur les cheveux et les cheveux sont ensuite rincés avec de l'eau et/ou un shampooing du commerce.

6. Utilisation d'un agent selon la revendication 3 pour matifier lors de l'éclaircissement et/ou le blondissement de fibres de kératine, en particulier de cheveux humains.
